# EUROPÄISCHE PATENTSCHRIFT

(11) **EP 3 650 840 B1**
(45) Veröffentlichungstag und Bekanntmachung des Hinweises auf die Patenterteilung: **10.07.2024**
(21) Anmeldenummer: 19207853.3
(22) Anmeldetag: 08.11.2019
(51) Int. Cl.: G01N 21/3504, G01N 21/39, G01N 33/14

(54) **LABOR-GASMESSGERÄT**
LABORATORY GAS MEASURING DEVICE
APPAREIL DE MESURE DES GAZ DE LABORATOIRE

(30) Priorität: 09.11.2018 AT 509622018
(43) Veröffentlichungstag der Anmeldung: 13.05.2020
(73) Patentinhaber: ACM-Automatisierung, Computertechnik, Meß- und Regeltechnik GmbH, 1140 Wien (AT)
(72) Erfinder: HARRAUER, Eduard, 1140 Wien (AT)
(74) Vertreter: SONN Patentanwälte GmbH & Co KG

(56) Entgegenhaltungen:
- CN-A- 106 442 322
- US-A- 4 101 383
- US-A- 5 155 019
- US-A1- 2018 188 164

## Beschreibung

Die Erfindung betrifft eine Vorrichtung zur Ermittlung der Konzentration eines Gases in einem Behälter, mit einer Halterung zur Aufnahme eines Behälters und mit einem optischen System, wobei das optische System eine im Wesentlichen monochromatische Lichtquelle zum Abstrahlen von Licht in einen in der Halterung aufgenommenen Behälter entlang einer optischen Achse und einen Lichtsensor zur Messung der Intensität des aus dem Behälter austretenden Lichts umfasst. Außerdem betrifft die Erfindung ein Verfahren zur Ermittlung der Konzentration eines Gases in einem Behälter mit einem optischen System, wobei das optische System eine im Wesentlichen monochromatische Lichtquelle zum Abstrahlen von Licht in einen Behälter entlang einer optischen Achse und einen Lichtsensor zur Messung der Intensität des aus dem Behälter austretenden Lichts umfasst, wobei mit dem optischen System mindestens zwei Messungen durchgeführt werden, wobei die Lage der optischen Achse in Bezug auf den Behälter zwischen den Messungen geändert wird.

Die DE 10 2014 112 969 A1 zeigt eine Vorrichtung und ein Verfahren zur Ermittlung einer Geometrie eines Behältnisses zur Verpackung eines Mediums mit einem Laser mit variierbarer Wellenlänge und einem Detektor. Zusätzlich kann im Halsbereich eine Kohlenstoffdioxidkonzentration gemessen werden, wobei das Behältnis zwischen den Messungen um 60° um seine Längsachse gedreht wird. Die Drehung des Behältnisses ist für die Ermittlung der Geometrie erforderlich, beeinflusst allerdings die Probe zwischen den Messungen, sodass mehrere Messungen zu unterschiedlichen Ergebnissen führen können.

Die WO 2012/001633 A2 zeigt eine Vorrichtung und ein Verfahren der eingangs angeführten Art. Die Lichtquelle und der Lichtsensor sind vertikal verstellbar. Zur Verminderung des Einflusses von Interferenzen entlang der optischen Achse kann das optische System mit einem Vibrationsmotor verbunden sein, sodass die optische Pfadlänge mechanisch moduliert wird. Der Einfluss der Vibration kann allerdings wellenlängenabhängig sein, sodass aufeinanderfolgende Messungen zu unterschiedlichen Messergebnissen führen können, weil die Vibration während der Wellenlängenmodulation der Lichtquelle zu unterschiedlichen optischen Pfaden für dieselbe Wellenlänge führt.

Die WO 2016/051341 A1 zeigt eine Apparatur, die vertikal verschoben werden kann, offenbar zur Anpassung an verschiedene Flaschenhöhen.

Die EP 3 364 170 A1 zeigt ein unbewegliches optisches System.

Die EP 2 620 761 A1 betrifft eine in-line CO2-Messung, die an Flaschen auf einem Förderband eingesetzt werden kann.

Auch die WO 2015/055743 A1 betrifft die Messung der CO2-Konzentration in einen Kopfbereich einer Flasche. Das optische System wird dabei mittels einer Zwinge auf einer Flaschenmündung fixiert.

Die US 4,101,383 A zeigt ein Differential-Lichtstreuungsphotometer zum Testen von Bakterienproben in Suspension. Dabei dreht sich ein Detektor in einem Bogen um jede Probe, um die Streulichtintensität als Funktion des Winkels relativ zum einfallenden Laserstrahl zu messen. Auf diese Weise wird ein winkelabhängiges Streumuster der Probe ermittelt.

In US 2018/188164 A1 ist ein System und Verfahren zum Messen einer Konzentration eines Gases in einem Behälter mit mindestens einer flexiblen oder variablen Seite oder Wand gezeigt. Bei diesem System und Verfahren führen die Komponenten des optischen Systems eine Translation zur Ermittlung der optischen Pfadlänge aus.

Die US 5,155,019 A zeigt ein Verfahren und eine Vorrichtung zum Nachweis des Vorhandenseins einer biologischen Aktivität in einer Materialprobe.

Im Zusammenhang mit der zunehmenden Verwendung von recyceltem Glas als Behältermaterial kann eine generelle Verschlechterung der Glasqualität beobachtet werden. Daraus ergeben sich zusätzliche systematische Abweichungen, die die Genauigkeit von optischen Messungen beeinträchtigen. Beispielsweise bewirken lokale Einschlüsse und Inhomogenitäten des Behältermaterials, dass das Messergebnis von der relativen Position des Behälters in Bezug auf die optische Achse abhängt und wirken sich daher unabhängig von der Geometrie des Behälters negativ auf die Reproduzierbarkeit der Ermittlungen aus.

Es ist eine Aufgabe der Erfindung, die Reproduzierbarkeit der einzelnen Messungen und somit letztlich die Genauigkeit, mit der die Konzentration des Gases ermittelt werden kann, gegenüber den bekannten Verfahren zu verbessern.

Die erfindungsgemäße Vorrichtung der eingangs angeführten Art sieht vor, dass das optische System um eine quer zur optischen Achse angeordnete Schwenkachse schwenkbar gelagert ist. Dementsprechend sieht das erfindungsgemäße Verfahren der eingangs angeführten Art vor, dass das optische System um eine quer zur optischen Achse angeordnete Schwenkachse geschwenkt wird. Die Schwenkachse ist quer, d.h. nicht parallel zur optischen Achse. Die Vorrichtung bzw. das Verfahren können zur Ermittlung der Konzentration eines vorbestimmten Gases in einem geschlossenen Behälter eingerichtet sein. Die optische Achse kann durch einen Kopfbereich ("head space") einer in der Halterung aufgenommenen Flasche verlaufen. Die mindestens zwei Messungen sind beispielsweise Messungen eines Transmissionsspektrums. Die Lichtquelle und der Lichtsensor wären demnach zur Messung eines Transmissionsspektrums angeordnet. Die mindestens zwei Messungen werden bei der Ermittlung der Konzentration berücksichtigt.

Der Behälter (z.B. die Flasche) bleiben demnach während der Messungen in Ruhe. Dadurch wird der Einfluss der Messungen auf die Probe verringert und somit die Reproduzierbarkeit verbessert. Im Unterschied zu einer relativ unkontrollierten Vibration ändert sich während der Aufnahme der Spektren nur die Wellenlänge und nicht die Lage der optischen Achse in Bezug auf den Behälter, sodass eine bessere Reproduzierbarkeit erreicht wird. Die bessere Reproduzierbarkeit verringert zufällige Abweichungen und erlaubt eine präzisere Kompensation etwaiger systematischer Abweichungen und somit letztlich eine höhere mögliche Genauigkeit. Die mehreren Messungen können beispielsweise statistisch ausgewertet und z.B. gefiltert und/oder gemittelt werden. Die gegenständliche Erfindung ist unabhängig von der Auswertungsmethode.

Die Schwenkachse kann z.B. im Wesentlichen normal auf die optische Achse angeordnet sein. Dabei kann sie mit einer Längsachse oder Drehsymmetrieachse des Behälters zusammenfallen. Dies hat den Vorteil, dass alle Messungen in derselben Höhe eines Flaschenhalses durchgeführt werden, was die Reproduzierbarkeit weiter erhöht. Gemäß einem Ausführungsbeispiel ist die optische Achse im Betrieb horizontal und die Schwenkachse vertikal angeordnet.

Die Vorrichtung kann einen Antrieb aufweisen, der zum Schwenken des optischen Systems eingerichtet ist. Der Antrieb kann z.B. ein Servomotor sein, d.h. ein spezieller Elektromotor, der die Kontrolle der Winkelposition seiner Motorwelle sowie der Drehgeschwindigkeit und Beschleunigung erlaubt.

Das optische System kann eine Trägerplatte aufweisen, an der die Lichtquelle und der Lichtsensor angebracht sind, wobei der Grundriss mindestens eine Öffnung aufweist, an der der Behälter in einen Detektionsbereich zwischen die Lichtquelle und den Lichtsensor eingeführt werden kann. Die Trägerplatte weist einen zum teilweisen Umfassen eines Behälters eingerichteten Grundriss. Der Grundriss kann z.B. U-förmig sein. Die Öffnung kann in einer Grundstellung oder Aufnahmestellung mit einer korrespondierenden Öffnung der Halterung überlagert sein, sodass der Behälter beim Einführen in die Halterung zugleich in den Detektionsbereich eingeführt wird.

In diesem Zusammenhang kann die Trägerplatte einen ersten Zahnkranzabschnitt aufweisen, wobei der erste Zahnkranzabschnitt mit einem zweiten Zahnkranzabschnitt in Eingriff steht, der mit dem oben erwähnten Antrieb verbunden ist. Wird der zweite Zahnkranzabschnitt verschränkt, kann dadurch eine entsprechend dem Übersetzungsverhältnis zwischen den Zahnkranzabschnitten korrespondierende Verschwenkung des ersten Zahnkranzabschnitt erreicht werden, und somit des optischen Systems. Der zweite Zahnkranzabschnitt kann optional als vollständiger Zahnkranz bzw. als Zahnrad ausgebildet sein.

Die Trägerplatte kann relativ zur Halterung gleitend gelagert sein. Z.B. kann eine schmiermittelfreie Gleitlagerung vorgesehen sein. Eine solche Lagerung ist besonders einfach und zuverlässig und weist zugleich eine hohe Laufruhe auf, sodass Störungen einer während einer Bewegung durchgeführten Messung möglichst vermieden werden.

Im Zusammenhang mit dem gegenständlichen Verfahren können mit dem optischen System mindestens fünf Messungen durchgeführt werden. Eine höhere Anzahl von Messungen ermöglicht eine genauere statistische Auswertung und erhöht somit letztlich die Genauigkeit der ermittelten Konzentration. Es können im Rahmen der vorliegenden Erfindung selbstverständlich auch deutlich mehr als fünf Messungen für eine Ermittlung verwendet werden, beispielsweise mindestens zehn Messungen, mindestens 15 Messungen, oder zwischen 20 und 60 Messungen, insbesondere zwischen 30 und 50 Messungen.

Gemäß einem speziellen Ausführungsbeispiel kann das optische System innerhalb eines Winkelbereichs von höchstens 180° geschwenkt werden. Es kann auch ein kleinerer Winkelbereich zum Schwenken ausreichen, z.B. innerhalb eines Winkelbereichs von weniger als 60°, oder innerhalb eines Winkelbereichs zwischen 5° und 30°, oder innerhalb eines Winkelbereichs zwischen 10° und 20°, oder innerhalb eines Winkelbereichs von 15°. Die genannten Winkelbereiche grenzen den Schwenkwinkel zwischen den äußersten Positionen des optischen Systems ein. Werden beispielsweise bei einer Ermittlung der Konzentration vom optischen System (d.h. auch der optischen Achse) Positionen innerhalb von -6° bis +6° relativ zu einer Grundstellung überstrichen oder eingenommen, entspricht dies einem Winkelbereich von 12°, welcher somit innerhalb des oben definierten Bereichs zwischen 10° und 20° liegt. Der verwendete Winkelbereich kann beispielsweise vorbestimmt sein.

Die Bewegung des optischen Systems innerhalb des Winkelbereichs kann zwischen drei und zehn Stopps bei unterschiedlichen Schwenkwinkeln umfassen. Beispielsweise können vier oder fünf Stopps vorgesehen sein. Die verschiedenen Stopps entsprechen unterschiedlichen Positionseinstellungen, an denen die Schwenkbewegung kurzzeitig unterbrochen wird. D.h. jeder Stopp entspricht einer Unterbrechung oder vorläufigen Einstellung. Die Stopps dienen dazu, jeweils mindestens eine Messung bei ruhigem optischem System durchführen zu können. Es kann optional zusätzliche Stopps ohne eigene Messung geben.

In diesem Zusammenhang können an mindestens einem Stopp, optional an jedem der zwischen drei und zehn Stopps, mindestens zwei Messungen durchgeführt werden. Aufgrund der verschiedenen äußeren Einflüsse auf das Messergebnis ist es nicht erforderlich, für jede einzelne Messung einen eigenen Stopp bei einem unterschiedlichen Schwenkwinkel vorzusehen.

Um Einflüsse seitens des Antriebs auf die Messungen ausschließen zu können, kann der Antrieb zur Bewegung des optischen Systems (z.B. ein Servomotor) während der Messung oder der Messungen ausgeschaltet werden. So kann beispielsweise eine Vibration der optischen Achse wegen eines "brummenden" Antriebs vermieden werden.

Darüber hinaus können zwischen den Stopps zusätzliche Messungen in Bewegung des optischen Systems durchgeführt werden. Diese zusätzlichen Messungen haben aus den oben erwähnten Gründen eine geringere Reproduzierbarkeit als die an einem Stopp durchgeführten Messungen. Dies kann bei der Auswertung der Messungen durch entsprechende Fehlerannahmen (und z.B. geringere Gewichtung) berücksichtigt werden. Nichtsdestotrotz können diese zusätzlichen Messungen den Gesamtinformationsgehalt und somit die Genauigkeit der ermittelten Konzentration erhöhen, ohne den Vorgang zusätzlich zu verzögern.

Gemäß einer Ausführungsvariante kann die Halterung mit einem austauschbaren Einschubelement ausgebildet sein. Das Einschubelement ist beispielsweise als U-förmiges Einschubelement ausgebildet. Darüber hinaus kann ein Set von austauschbaren Einschubelementen bereitgestellt werden, wobei verschiedene Einschubelemente für unterschiedliche Flaschenhalsquerschnitte und Ringwulstformen und -höhen im Bereich einer Flaschenöffnung vorgesehen sind. Die gezeigte Vorrichtung kann somit durch Austausch des verwendeten Einschubelements an einen Flaschenhals eines zu untersuchenden Behälters angepasst werden, sodass eine korrekte und reproduzierbare Anordnung eines Kopfbereichs einer Flasche im Detektionsbereich des optischen Systems erzielt wird. Genauer kann durch geeignete Einschubelemente gewährleistet werden, dass die optische Achse in einer Grundstellung im Wesentlichen eine Längsachse und/oder Drehsymmetrieachse des Behälters kreuzt, sodass in weiterer Folge die Längsachse und/oder Drehsymmetrieachse auch beim Schwenken des optischen Systems von der optischen Achse gekreuzt wird und somit für sämtliche Messungen eine im Wesentlichen gleiche optische Pfadlänge erzielt wird.

Dementsprechend kann vorgesehen sein, dass vor der Durchführung der Messungen der Behälter mittels eines geeigneten Einschubelements in Bezug auf das optische System zentriert wird. Genauer kann mittels des Einschubelements erreicht werden, dass eine zentrale Längsachse des Behälters die optische Achse des optischen Systems kreuzt. Das Einschubelement dient dabei als Adapter für die Halterung des Behälters, zum Beispiel der Flasche. Im Einzelnen wird das Einschubelement vom Benutzer entsprechend der Eigenschaften des vorliegenden Behälters ausgewählt und eine Halterung für den Behälter damit entsprechend dem Behälter angepasst. Das Set kann dabei Einschubelemente für alle gängigen (je nach Land weitgehend standardisierten) Flaschenformen oder zumindest Flaschenhalsformen umfassen.

Die Erfindung wird nachfolgend anhand von besonders bevorzugten Ausführungsbeispielen, auf die sie jedoch nicht beschränkt sein soll, und unter Bezugnahme auf die Zeichnungen noch weiter erläutert. Dabei zeigen im Einzelnen:
Fig. 1 schematisch eine Vorrichtung zur Ermittlung der Konzentration eines Gases in einem Behälter mit einer darin gehaltenen Flasche in einer schaubildlichen Ansicht;
Fig. 2 schematisch eine Draufsicht auf eine Messplattform der Vorrichtung gemäß Fig. 1;
Fig. 3 schematisch einen vertikalen Schnitt der Messplattform gemäß Fig. 1 gemäß der Linie III-III in Fig. 2;
Fig. 4 schematisch einen horizontalen Schnitt der Vorrichtung gemäß Fig. 1 gemäß der Linie IV-IV in Fig. 3;
Fig. 5 schematisch eine Seitenansicht auf eine Vorderseite der Messplattform gemäß Fig. 1 in einer Grundstellung der optischen Achse;
Fig. 6 schematisch einen horizontalen Schnitt der Vorrichtung gemäß Fig. 5 gemäß der Linie VI-VI;
Fig. 7 schematisch eine Seitenansicht auf eine Vorderseite der Messplattform gemäß Fig. 1 mit einer verschwenkten optischen Achse;
Fig. 8 schematisch einen horizontalen Schnitt der Vorrichtung gemäß Fig. 7 gemäß der Linie VIII-VIII;
Fig. 9a-9c schematisch drei verschiedene Einschubelemente zur Verwendung in der Vorrichtung gemäß Fig. 1 in einer schaubildlichen Ansicht; und
Fig. 10a-10c schematisch Draufsichten der drei Einschubelemente gemäß den Figuren 9a-9c;

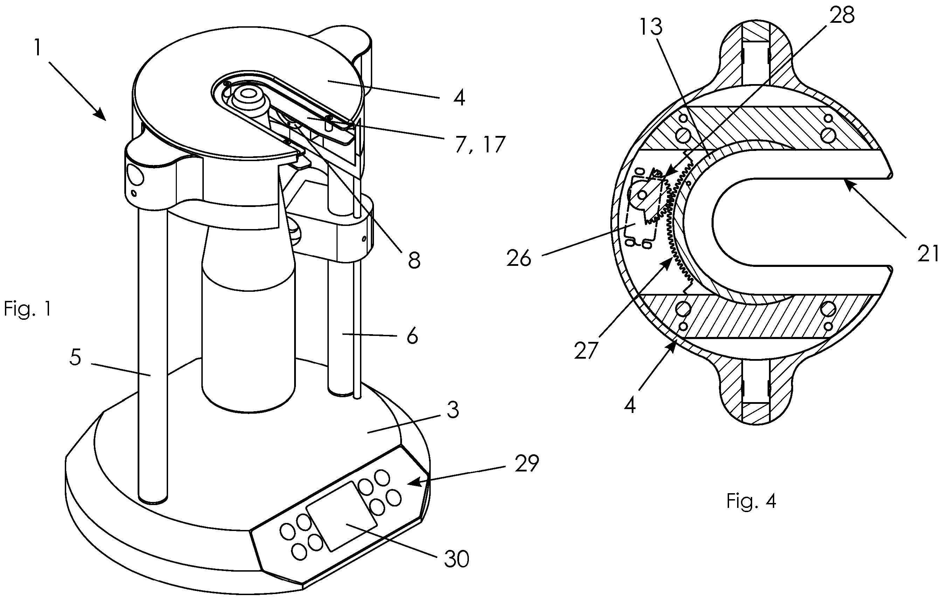

Die Figuren 1 bis 3 zeigen eine Vorrichtung 1 zur Ermittlung der Konzentration eines Gases in einem Behälter 2, hier einer Glasflasche. Die Vorrichtung 1 umfasst einen Sockel 3 und eine Messplattform 4, die über zwei Stützen 5, 6 mit dem Sockel 3 verbunden ist. Die Messplattform 4 weist eine Halterung 7 zur Aufnahme des Behälters 2 und ein optisches System 8 auf. Das optische System 8 umfasst eine im Wesentlichen monochromatische Lichtquelle 9 zum Abstrahlen von Licht in einen in der Halterung 7 aufgenommenen Behälter 2 entlang einer optischen Achse 10 und einen Lichtsensor 11 zur Messung der Intensität des aus dem Behälter 2 auf eine der Lichtquelle 9 gegenüberliegenden Seite austretenden Lichts. Das optische System 8 ist um eine Schwenkachse 12 schwenkbar gelagert. Die Schwenkachse 12 fällt dabei mit einer zentralen Längsachse (in diesem Beispiel zugleich eine Drehsymmetrieachse) des Behälters 2 zusammen und ist normal zur optischen Achse 10 angeordnet.

Das optische System 8 umfasst eine Trägerplatte 13, an der die Lichtquelle 9 (z.B. ein stimmbarer Dioden-Laser mit einer DFB (distributed feedback) Diode) und der Lichtsensor 11 angebracht sind. Lichtquelle 9 und Lichtsensor 11 sind hier nur schematisch dargestellt; geeignete Einrichtungen sind dem Fachmann geläufig, weshalb an dieser Stelle auf eine detaillierte technische Beschreibung verzichtet wird. Die Trägerplatte 13 hat einen zum teilweisen Umfassen eines Behälters 2 eingerichteten Grundriss, der im dargestellten Beispiel im Wesentlichen einem Segment eines Kreisringes entspricht. Die Trägerplatte 13 ist relativ zur Halterung 7 gleitend in einer korrespondierenden Führung 14 oder Schiene gelagert. Im Einzelnen weist die Trägerplatte 13 an einer radialen Außenseite eine entlang des Kreissegments umlaufende Nut 15 auf, in die eine korrespondierende Führungsleiste 16 mit einer im Wesentlichen ebenfalls kreissegmentförmigen Innenseite eingreift und die Trägerplatte 13 in vertikale Richtung festlegt.

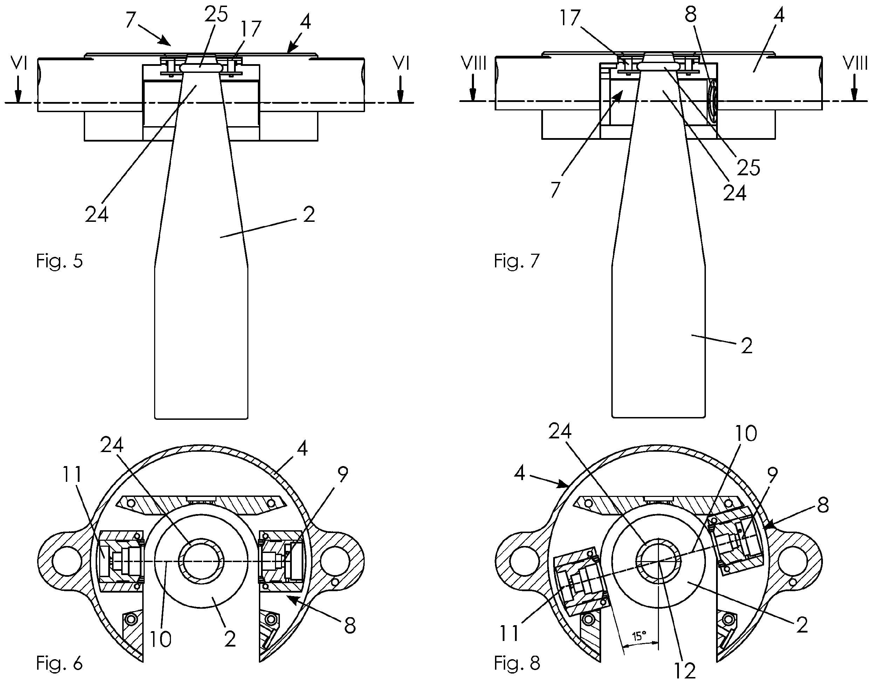

Die Halterung 7 der Messplattform 4 ist mit einem austauschbaren Einschubelement 17 ausgebildet. Das Einschubelement 17 ist aus zwei im Wesentlichen U-förmigen Platten 18, 19 gebildet, die in Verwendung in vertikale Richtung versetzt angeordnet und durch drei Distanzringe 20 voneinander beanstandet verbunden sind. Eine obere Platte 18 des Einschubelements 17 weist einen mit einer Einschubaufnahme 21 der Halterung 7 korrespondierenden Außenrand auf, sodass das Einschubelement 17 mit der oberen Platte 18 in eine U-förmige Nut 22 der Einschubaufnahme 21 aufgenommen werden kann. Der Innenrand 23 der unteren Platte 19 ist auf den Durchmesser eines Flaschenhalses 24 eines mit der Vorrichtung 1 zu verwendenden Flaschentyps abgestimmt. Beispielsweise ist der Innenrand 23 der unteren Platte 19 so dimensioniert, dass ein Ringwulst 25 des Flaschenhalses 24 auf der unteren Platte 19 aufliegt, wenn der Flaschenhals 24 in die Öffnung der unteren Platte 19 aufgenommen ist. Die Länge der Distanzringe 20 kann sich zwischen den einzelnen Einschubelementen 17 (siehe Figuren 5a-c und 6a-c) unterscheiden und dient dazu, den Abstand zwischen der unteren Platte 19 und der optischen Achse 10 festzulegen, sodass die Halterung 7 nicht nur in einer horizontalen Ebene sondern auch in vertikale Richtung eine geeignete Anordnung des Behälters 2 relativ zur optischen Achse 10 erzielt. Beispielsweise kann vorgesehen sein, dass die optische Achse bei einem Drittel der Höhe des Kopfbereichs oberhalb eines Flüssigkeitsspiegels im Behälter liegen soll. Aufgrund der unterschiedlichen Dichte der im Kopfbereich aufgenommenen Gaskomponenten ist die Reproduzierbarkeit der vertikalen Anordnung wichtig für eine hohe Genauigkeit der Ermittlung der Gaskonzentration.

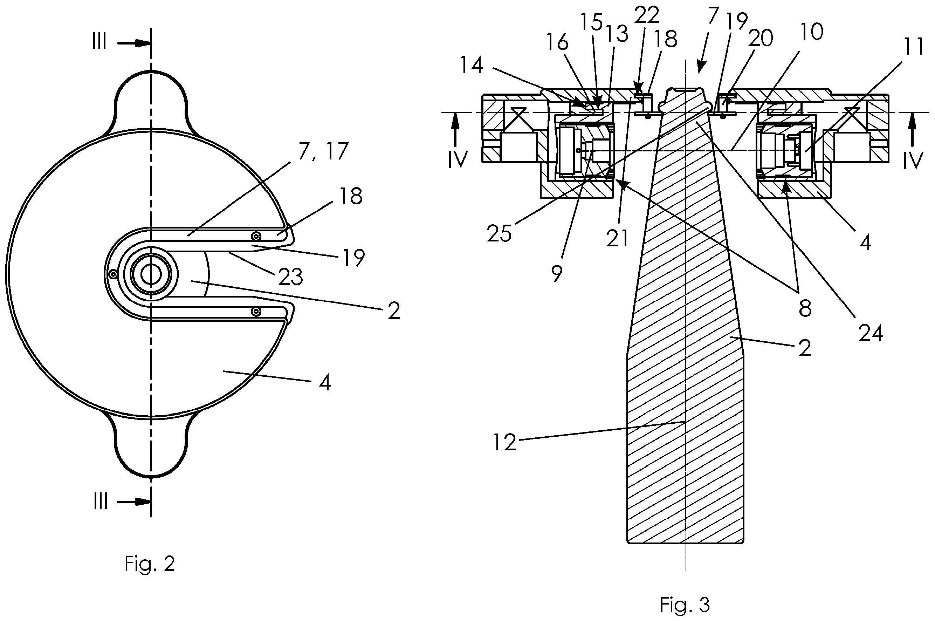

In Fig. 4 ist die Vorrichtung ohne das austauschbare Einschubelement 17 und ohne einen darin aufgenommenen Behälter 2 dargestellt. Wie hier ersichtlich ist, weist die Vorrichtung 1 einen Antrieb 26 in Form eines Servomotors auf, der zum Schwenken des optischen Systems 8 eingerichtet ist. An der Trägerplatte 13 ist ein erster Zahnkranzabschnitt 27 vorgesehen, der mit einem zweiten Zahnkranzabschnitt 28 in Eingriff steht. Der zweite Zahnkranzabschnitt 28 ist mit dem Antrieb 26 verbunden, sodass eine vom Antrieb 26 auf den zweiten Zahnkranzabschnitt 28 aufgebrachtes Drehmoment zu einer Schwenkbewegung des optischen Systems 8 führt. Der Antrieb 26 ist in Fig. 4 nur gestrichelt eingezeichnet, weil er sich in der in Fig. 3 gezeigten Ansicht unterhalb der Schnittebene IV-IV befindet und daher in der in Fig. 4 gezeigten Ansicht von unten auf die Schnittebene "oberhalb" der Zeichnungsebene befinden würde und nicht sichtbar wäre.

Das optische System 8 und der Antrieb 26 sind mit einer Steuerungselektronik im Sockel 3 elektrisch verbunden, sodass der Antrieb 26 und die Lichtquelle 9 von der Steuerungselektronik angesteuert werden der Lichtsensor 11 ausgelesen werden kann.

Zur Ermittlung der Konzentration eines Gases im Kopfbereich einer geschlossenen Getränkeflasche mittels der hier gezeigten Vorrichtung würde ein Benutzer vor der Durchführung der Messungen ein zur Getränkeflaschen passendes Einschubelement 17 auswählen und in die Einschubaufnahme 21 einführen. Anschließend kann die Getränkeflasche in das Einschubelement 17 eingeführt und somit in der Halterung 7 aufgenommen, genauer aufgehängt, werden. Auf diese Weise ist die Getränkeflasche reproduzierbar in der Vorrichtung 1 positioniert. Anschließend wählt der Benutzer auf einem Eingabefeld 29 am Sockel 3 das gewünschte Messprogramm aus, beispielsweise das Messprogramm zur Ermittlung der Konzentration von CO2 im Kopfbereich der Getränkeflasche.

Entsprechend der Benutzereingaben wird dann das Messverfahren von der Steuerungselektronik durchgeführt und abschließend die ermittelte Konzentration auf dem Display 30 am Sockel 3 ausgegeben. Im Einzelnen läuft das Messverfahren gemäß dem vorliegenden Ausführungsbeispiel folgendermaßen ab: das optische System 8 wird aus der in den Figuren 1-4 und insbesondere Figuren 5 und 6 gezeigten Grundstellung 15° gegen den Uhrzeigersinn zu einem ersten Stopp geschwenkt (oder gedreht). Dazu wird der Antrieb 26 eingeschaltet und für eine dem gewünschten Schwenkwinkel entsprechende Bewegung angesteuert. Die so erreichte Stellung, in der die optische Achse 10 um 15° gegen den Uhrzeigersinn um die Schwenkachse 12 aus der Grundstellung geschwenkt ist, ist in den Figuren 7 und 8 dargestellt. Am ersten Stopp wird der Antrieb 26 ausgeschaltet und es werden fünf Messungen eines Transmissionsspektrums aufgenommen, wobei jeweils die Wellenlänge der Lichtquelle 9 über einen vorgegebenen Wellenlängenbereich (typischerweise mit einer Breite von einigen Nanometern) kontinuierlich variiert und die vom Lichtsensor 11 währenddessen empfangene Lichtintensität aufgezeichnet wird. Nach der Aufnahme der fünften Messung wird der Antrieb 26 wieder eingeschaltet für einen Schwenkwinkel von 7,5° im Uhrzeigersinn gemäß einer vorgegebenen Drehgeschwindigkeit angesteuert. Nach jedem einzelnen Winkelgrad (Drehung um 1°) wird eine neuerliche Messung eines Transmissionsspektrums ausgelöst. Sobald die Schwenkbewegung um 7,5° abgeschlossen ist und somit der zweite Stopp erreicht ist, wird der Antrieb 26 wieder ausgeschaltet und es werden erneut fünf Messungen eines Transmissionsspektrums aufgenommen. Dieser Vorgang wiederholt sich für drei weitere Schwenkbewegungen um jeweils 7,5° im Uhrzeigersinn bis am fünften Stopp fünf Messungen durchgeführt wurden. Zu diesem Zeitpunkt ist die optische Achse 10 um 15° im Uhrzeigersinn aus der Grundstellung geschwenkt. Am Ende liegen insgesamt 25 in Ruhe aufgenommene Transmissionsspektren und 28 (4x7) in Bewegung aufgenommene Transmissionsspektren vor. Anschließend der Antrieb 26 eingeschaltet das optische System 8 fährt zurück in die Grundstellung, sodass die Getränkeflasche aus der Vorrichtung 1 entnommen werden kann. Parallel dazu wird auf Basis der aufgenommenen insgesamt 53 Transmissionsspektren die CO2-Konzentration in der Getränkeflasche ermittelt und nach Abschluss der Ermittlung am Display 30 angezeigt. Methoden zur Ermittlung einer Gaskonzentration aus einem Transmissionsspektrum sind hinlänglich bekannt. Diese basieren beispielsweise auf einer Auswertung der relativen Intensität der festgestellten Absorption bei den Wellenlängen der bekannten Absorptionslinien des gesuchten Gases. Es kann auf dieser Grundlage aus jedem einzelnen Transmissionsspektrum eine Konzentration ermittelt werden und anschließend die erhaltenen Konzentrationen gefiltert (beispielsweise Ausscheiden von Ausreißern) und gemittelt werden.

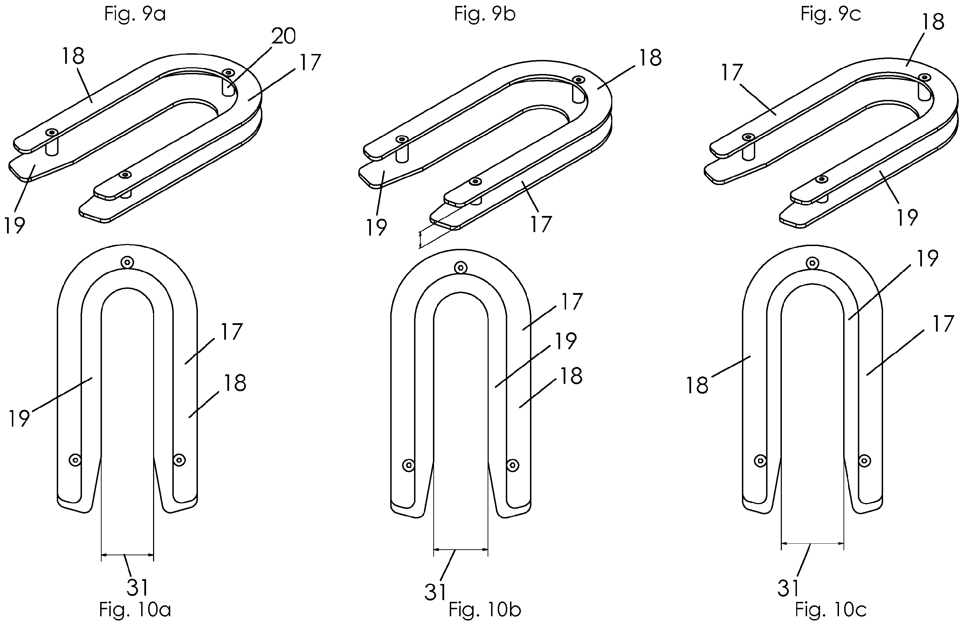

In den Figuren 9a bis 9c sowie 10a bis 10c sind drei verschiedene Einschubelemente 17 dargestellt. Jedes Einschubelement 17 hat eine obere Platte 18 und eine untere Platte 19, die jeweils durch drei Distanzringe 20 verbunden sind. Die drei zeigten Einschubelemente 17 unterscheiden sich im Wesentlichen durch die Breite 31 der Öffnung der unteren Platte 19, welche auf den Durchmesser eines aufzunehmenden Flaschenhalses abgestimmt ist. Bei dem in den Figuren 9a und 10a dargestellten Einschubelement 17 ist diese Breite 26 mm, für einen Flaschenhalsdurchmesser von zwischen 25,5-26 mm. Bei dem in den Figuren 9b und 10b dargestellten Einschubelement 17 ist diese Breite 27 mm, für einen Flaschenhalsdurchmesser von zwischen 26,5-27 mm. Und bei dem in den Figuren 9c und 10c dargestellten Einschubelement 17 ist diese Breite 31 mm, für einen Flaschenhalsdurchmesser von zwischen 30,5-31 mm.

## Patentansprüche

1. Vorrichtung (1) zur Ermittlung der Konzentration eines Gases in einem Behälter (2), mit einer Halterung (7) zur Aufnahme eines Behälters (2) und mit einem optischen System (8), wobei das optische System (8) eine im Wesentlichen monochromatische Lichtquelle (9) zum Abstrahlen von Licht in einen in der Halterung (7) aufgenommenen Behälter (2) entlang einer optischen Achse (10) und einen Lichtsensor (11) zur Messung der Intensität des aus dem Behälter (2) austretenden Lichts umfasst, **dadurch gekennzeichnet, dass** das optische System (8) um eine quer zur optischen Achse (10) angeordnete Schwenkachse (12) schwenkbar gelagert ist.

2. Vorrichtung (1) nach Anspruch 1, **dadurch gekennzeichnet, dass** die Lichtquelle (9) und der Lichtsensor (11) zur Messung eines Transmissionsspektrums angeordnet sind.

3. Vorrichtung (1) nach Anspruch 1 oder 2, **dadurch gekennzeichnet, dass** die Schwenkachse (12) im Wesentlichen normal auf die optische Achse (10) angeordnet ist.

4. Vorrichtung (1) nach einem der Ansprüche 1 bis 3, **dadurch gekennzeichnet, dass** die Vorrichtung (1) einen Antrieb (26) aufweist, der zum Schwenken des optischen Systems (8) eingerichtet ist.

5. Vorrichtung (1) nach Anspruch 4, **dadurch gekennzeichnet, dass** das optische System (8) eine Trägerplatte (13) aufweist, an der die Lichtquelle (9) und der Lichtsensor (11) angebracht sind, wobei der Grundriss mindestens eine Öffnung aufweist, an der der Behälter in einen Detektionsbereich zwischen die Lichtquelle und den Lichtsensor eingeführt werden kann.

6. Vorrichtung (1) nach Anspruch 5, **dadurch gekennzeichnet, dass** die Trägerplatte (13) einen ersten Zahnkranzabschnitt (27) aufweist, wobei der erste Zahnkranzabschnitt (27) mit einem zweiten Zahnkranzabschnitt (28) in Eingriff steht, der mit dem Antrieb (26) verbunden ist.

7. Vorrichtung (1) nach einem der Ansprüche 1 bis 6, **dadurch gekennzeichnet, dass** die Halterung (7) mit einem austauschbaren Einschubelement (17) ausgebildet ist.

8. Verfahren zur Ermittlung der Konzentration eines Gases in einem Behälter (2) mit einem optischen System (2), wobei das optische System (8) eine im Wesentlichen monochromatische Lichtquelle (9) zum Abstrahlen von Licht in einen Behälter (2) entlang einer optischen Achse (10) und einen Lichtsensor (11) zur Messung der Intensität des aus dem Behälter (2) austretenden Lichts umfasst, wobei mit dem optischen System (8) mindestens zwei Messungen durchgeführt werden, wobei die Lage der optischen Achse (10) in Bezug auf den Behälter (2) zwischen den Messungen geändert wird, **dadurch gekennzeichnet, dass** das optische System um eine quer zur optischen Achse (10) angeordnete Schwenkachse (12) geschwenkt wird.

9. Verfahren nach Anspruch 8, **dadurch gekennzeichnet, dass** die mindestens zwei Messungen Messungen eines Transmissionsspektrums sind.

10. Verfahren nach Anspruch 8 oder 9, **dadurch gekennzeichnet, dass** mit dem optischen System (8) mindestens fünf Messungen durchgeführt werden.

11. Verfahren nach einem der Ansprüche 8 bis 10, **dadurch gekennzeichnet, dass** das optische System (8) innerhalb eines Winkelbereichs von höchstens 180° geschwenkt wird, vorzugsweise innerhalb eines Winkelbereichs von weniger als 60°, insbesondere innerhalb eines Winkelbereichs zwischen 5° und 30°, insbesondere innerhalb eines Winkelbereichs zwischen 10° und 20°, insbesondere innerhalb eines Winkelbereichs von 15°.

12. Verfahren nach Anspruch 11, **dadurch gekennzeichnet, dass** die Bewegung des optischen Systems (8) innerhalb des Winkelbereichs zwischen drei und zehn Stopps bei unterschiedlichen Schwenkwinkeln umfasst, vorzugsweise vier oder fünf Stopps.

13. Verfahren nach Anspruch 12, **dadurch gekennzeichnet, dass** an mindestens einem Stopp, vorzugsweise an jedem Stopp, mindestens zwei Messungen durchgeführt werden.

14. Verfahren nach Anspruch 12 oder 13, **dadurch gekennzeichnet, dass** ein Antrieb (26) zur Bewegung des optischen Systems (8) während der Messung oder während der Messungen ausgeschaltet wird.

15. Verfahren nach einem der Ansprüche 8 bis 14, **dadurch gekennzeichnet, dass** vor der Durchführung der Messungen der Behälter (2) mittels eines geeigneten Einschubelements (17) in Bezug auf das optische System (8) zentriert wird.

## Claims

1. Device (1) for determining the concentration of a gas in a container (2), having a holder (7) for receiving a container (2) and having an optical system (8), the optical system (8) comprising a substantially monochromatic light source (9) for emitting light into a container (2) received in the holder (7) along an optical axis (10) and comprising a light sensor (11) for measuring the intensity of the light emerging from the container (2), **characterised in that** the optical system (8) is pivotally mounted about a pivot axis (12) arranged transversely to the optical axis (10).

2. Device (1) according to claim 1, **characterised in that** the light source (9) and the light sensor (11) are arranged to measure a transmission spectrum.

3. Device (1) according to either claim 1 or claim 2, **characterised in that** the pivot axis (12) is substantially normal to the optical axis (10).

4. Device (1) according to any of claims 1 to 3, **characterised in that** the device (1) has a drive (26) which is designed to pivot the optical system (8).

5. Device (1) according to claim 4, **characterised in that** the optical system (8) has a carrier plate (13) to which the light source (9) and the light sensor (11) are attached, the base having at least one opening at which the container can be introduced into a detection region between the light source and the light sensor.

6. Device (1) according to claim 5, **characterised in that** the carrier plate (13) has a first ring gear portion (27), the first ring gear portion (27) engaging with a second ring gear portion (28) which is connected to the drive (26).

7. Device (1) according to any of claims 1 to 6, **characterised in that** the holder (7) is formed having an exchangeable insertion element (17).

8. Method for determining the concentration of a gas in a container (2) using an optical system (2), the optical system (8) comprising a substantially monochromatic light source (9) for emitting light into a container (2) along an optical axis (10) and comprising a light sensor (11) for measuring the intensity of the light emerging from the container (2), at least two measurements being carried out using the optical system (8), the position of the optical axis (10) in relation to the container (2) being changed between the measurements, **characterised in that** the optical system is pivoted about a pivot axis (12) arranged transversely to the optical axis (10).

9. Method according to claim 8, **characterised in that** the at least two measurements are measurements of a transmission spectrum.

10. Method according to either claim 8 or claim 9, **characterised in that** at least five measurements are carried out using the optical system (8).

11. Method according to any of claims 8 to 10, **characterised in that** the optical system (8) is pivoted within an angular range of at most 180°, preferably within an angular range of less than 60°, in particular within an angular range of between 5° and 30°, in particular within an angular range of between 10° and 20°, in particular within an angular range of 15°.

12. Method according to claim 11, **characterised in that** the movement of the optical system (8) within the angular range comprises between three and ten stops at different pivot angles, preferably four or five stops.

13. Method according to claim 12, **characterised in that** at least two measurements are carried out at at least one stop, preferably at each stop.

14. Method according to either claim 12 or claim 13, **characterised in that** a drive (26) for moving the optical system (8) is switched off during the measurement or during the measurements.

15. Method according to any of claims 8 to 14, **characterised in that** before carrying out the measurements, the container (2) is centred with respect to the optical system (8) by means of a suitable insertion element (17).

## Revendications

1. Dispositif (1) pour la détermination de la concentration d'un gaz dans un contenant (2), avec un élément de retenue (7) pour la réception d'un contenant (2) et avec un système optique (8), dans lequel le système optique (8) comprend une source de lumière (9) sensiblement monochromatique pour l'émission d'une lumière dans un contenant (2) reçu dans l'élément de retenue (7) le long d'un axe optique (10) et un capteur de lumière (11) pour la mesure de l'intensité de la lumière sortant du contenant (2), **caractérisé en ce que** le système optique (8) est monté de manière à pouvoir pivoter autour d'un axe de pivotement (12) disposé transversalement par rapport à l'axe optique (10).

2. Dispositif (1) selon la revendication 1, **caractérisé en ce que** la source de lumière (9) et le capteur de lumière (11) sont disposés pour la mesure d'un spectre de transmission.

3. Dispositif (1) selon la revendication 1 ou 2, **caractérisé en ce que** l'axe de pivotement (12) est disposé sensiblement perpendiculairement à l'axe optique (10).

4. Dispositif (1) selon l'une quelconque des revendications 1 à 3, **caractérisé en ce que** le dispositif (1) présente un entraînement (26), qui est conçu pour faire pivoter le système optique (8).

5. Dispositif (1) selon la revendication 4, **caractérisé en ce que** le système optique (8) présente une plaque de support (13), sur laquelle la source de lumière (9) et le capteur de lumière (11) sont fixés, dans lequel la projection horizontale présente au moins une ouverture, sur laquelle le contenant peut être introduit dans une zone de détection entre la source de lumière et le capteur de lumière.

6. Dispositif (1) selon la revendication 5, **caractérisé en ce que** la plaque de support (13) présente une première partie de couronne dentée (27), dans lequel la première partie de couronne dentée (27) est en prise avec une deuxième partie de couronne dentée (28), qui est reliée à l'entraînement (26).

7. Dispositif (1) selon l'une quelconque des revendications 1 à 6, **caractérisé en ce que** l'élément de retenue (7) est réalisé avec un élément d'insertion (17) interchangeable.

8. Procédé pour la détermination de la concentration d'un gaz dans un contenant (2) avec un système optique (2), dans lequel le système optique (8) comprend une source de lumière (9) sensiblement monochromatique pour l'émission de lumière dans un contenant (2) le long d'un axe optique (10) et un capteur de lumière (11) pour la mesure de l'intensité de la lumière sortant du contenant (2), dans lequel au moins deux mesures sont mises en oeuvre avec le système optique (8), dans lequel la position de l'axe optique (10) par rapport au contenant (2) est modifiée entre les mesures, **caractérisé en ce que** le système optique est amené à pivoter autour d'un axe de pivotement (12) disposé transversalement par rapport à l'axe optique (10).

9. Procédé selon la revendication 8, **caractérisé en ce que** les au moins deux mesures sont des mesures d'un spectre de transmission.

10. Procédé selon la revendication 8 ou 9, **caractérisé en ce qu'**au moins cinq mesures sont mises en oeuvre avec le système optique (8).

11. Procédé selon l'une quelconque des revendications 8 à 10, **caractérisé en ce que** le système optique (8) est amené à pivoter à l'intérieur d'une plage angulaire de maximum 180°, de préférence à l'intérieur d'une plage angulaire de moins de 60°, en particulier à l'intérieur d'une plage angulaire comprise entre 5° et 30°, en particulier à l'intérieur d'une plage angulaire comprise entre 10° et 20°, en particulier à l'intérieur d'une plage angulaire de 15°.

12. Procédé selon la revendication 11, **caractérisé en ce que** le mouvement du système optique (8) à l'intérieur de la plage angulaire comprend entre trois et dix arrêts pour différents angles de pivotement, de préférence quatre ou cinq arrêts.

13. Procédé selon la revendication 12, **caractérisé en ce qu'**au moins deux mesures sont mises en oeuvre à au moins un arrêt, de préférence à chaque arrêt.

14. Procédé selon la revendication 12 ou 13, **caractérisé en ce qu'**un entraînement (26) pour le mouvement du système optique (8) est mis hors circuit pendant la mesure ou pendant les mesures.

15. Procédé selon l'une quelconque des revendications 8 à 14, **caractérisé en ce qu'**avant la mise en oeuvre des mesures le contenant (2) est centré par rapport au système optique (8) au moyen d'un élément d'insertion (17) adapté.
